**Europäisches Patentamt**

**European Patent Office**

**Office européen des brevets**

(19)

(11) Veröffentlichungsnummer: **0 232 791**
A2

## (12) EUROPÄISCHE PATENTANMELDUNG

(21) Anmeldenummer: **87101086.4**

(22) Anmeldetag: **27.01.87**

(51) Int. Cl.⁴: **A 61 F 2/30**

(30) Priorität: **12.02.86 DD 286973**

(43) Veröffentlichungstag der Anmeldung: **19.08.87**
**Patentblatt 87/34**

(84) Benannte Vertragsstaaten: **AT BE CH DE FR GB IT LI NL SE**

(71) Anmelder: **Technische Hochschule Karl-Marx-Stadt, Postfach 964, DDR-9010 Karl-Marx-Stadt (DD)**

(72) Erfinder: **Kurze, Peter, Doz.Dr.sc.nat., Hauptstrasse 48, DDR-9109 Oberlichtenau (DD)**
Erfinder: **Krysmann, Waldemar, Dr.rer.nat., Fritz-Schmenkel-Strasse 37, DDR-9071 Karl-Marx-Stadt (DD)**
Erfinder: **Knöfler, Wolfram, Dr.med., Kantstrasse 34, DDR-7030 Leipzig (DD)**
Erfinder: **Graf, Hans-Ludwig, Dr.med., Volksgartenstrasse 26/56, DDR-7024 Leipzig (DD)**

(74) Vertreter: **Zipse + Habersack, Kemnatenstrasse 49, D-8000 München 19 (DE)**

(54) **Keramisiertes Metallimplantat.**

(57) Durch Anwendung der anodischen Oxidation unter Funkenentladung in wässrigen Elektrolyten wird erfindungsgemäss resorbierbare Kalziumphosphatkeramik mit der Konzentration von 10–40% mit definiertem Konzentrationsverlauf in der Oxidschicht so eingebaut, dass an der dem Implantatbett zugewandten Seite das maximale Angebot vorliegt. Das Kalziumphosphatkeramik-Depot nimmt in Richtung der Phasengrenze Metall/Oxidschicht ab, so dass der Knochen in das keramisierte Metallimplantat hineinwächst und eine grosse Haftfestigkeit erreicht wird.

Vor der Keramisierung erhält das Metallimplantat durch eine mechanische und/oder chemische und/oder elektrochemische Vorbehandlung Strukturdimensionen von 100–400 µm, die die wirksame Oberfläche um mehr als 400% erhöht. Um gewebe- oder knochenspezifische Eigenschaften zu erzeugen, kann eine Nachbehandlung in speziellen Medien, wie z.B. Aminosäuren, Antibiotika, durchgeführt werden. Das keramisierte Metallimplantat findet in der Medizin als Hemi- und Endoprothese Anwendung.

Keramisiertes Metallimplantat

Die Erfindung betrifft ein keramisiertes Metallimplantat für die Verwendung als Hemi- und Endoprothese in der Medizin.

Bekannte Implantate sind aus Metallen oder -legierungen wie Titan, Tantal, Chrom-Kobalt-Molybdän-Legierungen als auch Materialien wie Kohlenstoff, Polymere oder Aluminiumoxidkeramik oder deren Kombinationen hergestellt. Eine Verbesserung der Implantateigenschaften wird auch durch gezielte Oberflächenbehandlung angestrebt. In der DE-OS 2 324 867 wird durch die Einlagerung von Ionen in die Oberfläche des metallischen Implantates kurzzeitig seine Biokompatibilität verbessert. Durch Diffusion gelangen aber schon nach kurzer Zeit nach dem Implantieren schädliche Ionen in das Implantatbett, und eine lange Liegedauer des Implantates ist damit nicht gewährleistet.

In der DE-OS 2 838 759 wird eine Passivierung der Implantatoberfläche mittels aufwendiger Vakuumtechnik angestrebt. Die so aufgebrachten Schichten sind sehr dünn und haften schlecht, und die Liegedauer des so hergestellten Implantates im Körper wird dadurch negativ beeinflußt. In der EP-A 0 023 608 ist eine bioreaktive Kalziumphosphatkeramik, die auf die Oberfläche des metallischen Implantatkörpers durch Druck-Sinter-Vorgänge aufgepreßt wird, beschrieben. In der EP-A 006 544 ist ein implantierbarer metallischer Knochenwerkstoff vorgestellt, auf dem kugelförmige Kalziumphosphatpartikel in die Oberfläche eingebracht sind. Das wird erreicht, indem an die Innenwandung der Gußform die Kalziumphosphatpartikel geklebt und die Form mit Metall ausgegossen wird. Das gleiche ist durch Heißpressen, Flamm- oder Plasmaspritzen erreichbar. Die so aufgebrachte Kalziumphosphatkeramik ist aber im Körper leicht auslaugbar und außerdem in ihrem Konzentrationsangebot dem Knochenwachstum unzureichend angepaßt, und notwendige Strukturdimensionen für das Einwachsen des Gewebes werden nicht beachtet. Die Liegedauer des Implantates wird damit negativ beeinflußt.

In der Literatur (Strauß, V. E. Bild der Wissenschaft 21 (1984), S. 110 - 122) ist ein Keramik-Metallimplantat-Verbund beschrieben. Die Vorzüge der Keramik sollen gleichzeitig mit der Festigkeit des Metalles kombiniert werden. Die aufgebrachten Keramikschichten haften aber unzureichend auf dem Metall. Dadurch ist das so einoperierte Implantat in seiner Liegedauer stark eingeschränkt.

Aufgabe der Erfindung ist es, ein Metallimplantat zu schaffen, welches eine vergrößerte Oberfläche gegenüber dem Implantatbett aufweist und das Knochenwachstum entsprechend dem Heilungs- und Wachstumsprozeß selbst regelt. Ferner soll das Metallimplantat von langer Liegedauer sein sowie eine hohe Gewebe- und Knochenfreundlichkeit aufweisen. Erfindungsgemäß wird die Aufgabe dadurch gelöst, daß ein Metallimplantat-Grundkörper aus Ventilmetall oder dessen Legierung oder aus einem mit Ventilmetall bzw. dessen Legierung überzogenes Metall mechanisch und/oder chemisch und/oder elektrochemisch definiert strukturiert wird, so daß eine Oberflächenrauheit von 100 µm bis 400 µm die wirksame Oberfläche um mehr als 400 % erhöht. Auf diesem Metallimplantat-Grundkörper ist eine Keramik mit einer

- 3 -

Dicke von 5 - 30 $\mu$m und Strukturdimensionen von 2 - 20 $\mu$m Durchmesser aufgebracht, die zu 100 % aus einer kurzzeiterschmolzenen arteigenen Oxidschicht aus nichtstöchiometrischen Oxiden und Mischoxiden oder aus 90 % bis 60 % dieser arteigenen Oxide und 10 % bis 40 % Kalziumphosphatkeramik besteht. Dabei liegt ein Konzentrationsverlauf der Kalziumphosphatkeramik bis nahe der Phasengrenze Metallimplantat-Grundkörper / Keramik < 1 $\mu$m vor, wobei das maximale Angebot der Kalziumphosphatkeramik an der dem Implantatbett zugewandten Seite anliegt.

Erfindungsgemäß bestimmt das definiert in der Oxidschicht angeordnete Kalziumphosphatkeramik-Depot den Heilungs- und Wachstumsprozeß und die Haftfestigkeit des Knochens am beschichteten Metallimplantat, da sich in der Phasengrenze der dem Metallimplantatgrundkörper zugewandten Seite der Oxidschicht in einem Bereich kleiner 1 $\mu$m keine resorbierbare Kalziumphosphat-Keramik befindet.

Erfindungsgemäß wächst durch den Resorbtionsprozeß der Knochen in das keramisierte Metallimplantat hinein und schafft die für die große Haftfähigkeit notwendigen Knochenkontakte, wobei sich die Knochenkontaktflächenbildung selbst über die Nutzung des Angebotes an resorbierbarer Kalziumphosphat-Keramik bei entsprechender Belastungsverteilung steuert. Damit bilden sich keine Stellen ohne Knochenkontakte aus.
Die Herstellung der keramisierten Metallimplantate erfolgt gewöhnlich in einem Einstufenprozeß. Um gewebe- oder knochenspezifische Eigenschaften zu erzeugen, kann eine Nachbehandlung in speziellen Medien, wie z. B. Aminosäuren, Antibiotika, durchgeführt werden.

- 4 -

Die Erfindung soll nachstehend in einem Ausführungsbeispiel näher erläutert werden.

Ein aus Titanium bestehendes Metallimplantat, z. B. eine Hüftgelenkendoprothese, wird einer mechanischen Strukturierung durch Strahlbehandlung mit Edelkorund einer Körnung von 100 bis 200 $\mu$m 5 Minuten unterzogen und danach einer chemischen Ätzung in einer 55 %igen Flußsäure-Lösung ausgesetzt. Oberflächentastschnitte und mikroskopische Messungen zeigen Strukturdimensionen von 150 bis 280 $\mu$m.

Die anschließende anodische Oxidation unter Funkenentladung in einer wäßrigen Lösung von komplexgebundenem Kalziumphosphat und dispergiertem Tri- und tetrakalziumphosphat führt unter Impulsspannungen zu einer 12 $\mu$m starken, kurzzeiterschmolzenen, arteigenen Oxidschicht aus nichtstöchiometrischen Oxiden und Mischoxiden, die Strukturdimensionen von 6 $\mu$m zeigen und an der dem Implantatbett zugewandten Seite einen Kalziumphosphatkeramik-Anteil von 27 % aufweisen.

Die erfindungsgemäß behandelten Implantate zeigen bei Implantation am oder im Knochen einen um ca. 15 % erhöhten Anteil an direkten Knochenkontakten.

- 5 -

Patentanspruch

Keramisiertes Metallimplantat, vorzugsweise unter Anwendung der anodischen Oxidation unter Funkenentladung in wäßrigen Elektrolyten, dadurch gekennzeichnet, daß auf einem strukturierten Metallimplantat-Grundkörper aus Ventilmetall mit einer Oberflächenrauheit von 100 - 400 µm eine Keramik mit einer Dicke von 5 - 30 µm und Strukturdimensionen von 2 - 20 µm Durchmesser aufgebracht ist, die zu 100 % aus einer kurzzeiterschmolzenen, arteigenen Oxidschicht aus nichtstöchiometrischen Oxiden und Mischoxiden oder aus 90 - 60 % dieser arteigenen Oxide und 10 - 40 % Kalziumphosphatkeramik besteht, wobei ein Konzentrationsverlauf der Kalziumphosphatkeramik bis nahe der Phasengrenze Metallimplantat-Grundkörper / Keramik <1 µm vorliegt und das maximale Angebot der Kalziumphosphatkeramik dem Implantatbett zugewandt ist.